# EUROPEAN PATENT SPECIFICATION

(11) **EP 0 587 541 B1**
(45) Date of publication and mention of the grant of the patent: **13.05.1998**
(21) Application number: 93830361.7
(22) Date of filing: 03.09.1993
(51) Int. Cl.: C07K 1/14, C07K 1/22, C12N 15/10

(54) **Process to purify the big-endothelin protein**
Verfahren zur Reinigung des Big-Endothelin Proteins
Procédé de purification de protéine Big-endothéline

(30) Priority: 09.09.1992 IT RM920658
(43) Date of publication of application: 16.03.1994
(73) Proprietor: TECNOGEN S.C.P.A., 81015 Piana de Monte Verna (Caserta) (IT)
(72) Inventor: Fassina, Giorgio, c/o TECNOGEN ScpA, I-81015 Piana di Monte Verna (CE) (IT); Merli, Silvia, c/o TECNOGEN ScpA, I-81015 Piana di Monte Verna (CE) (IT)
(74) Representative: de Simone, Domenico

(56) References cited:
- EP-A- 0 147 819
- WO-A-90/02179
- THE JOURNAL OF BIOCHEMISTRY, vol. 110, no. 4, October 1991, H. OHASHI et al., pp. 628-634

## Description

This invention relates to a process to purify the Big-Endothelin (Big-ET) protein from crude extracts having proteolytic activities, with high final product yield, by means of trypsin digestion and affinity chromatography procedures, using a Big-ET specific peptide ligand, stable to trypsin.

The process allows to recover high purity recombinant Big-ET from host cells extracts, thus avoiding expensive either liquid or solid phase chemical synthesis procedures.

It is known in the art that endothelin (ET) is a 21 amino acid peptide, having a powerful vasoconstrictive activity (Yanagisawa and al., Nature, 332,411-415, 1988). ET is synthesized by endothelial cells as a 200 amino acid precursor (pre-pro-ET),then converted to Big-ET (38 amino acids) and finally to ET (21 amino acids) by proteolytic digestion. Enzyme(s) responsible for said reactions is (are) not yet fully characterized and is (are) known as ECE. Big-ET is not active; however it may be activated, both *in vitro* and *in vivo,* also by large spectrum proteolytic enzymes, such as chymotrypsin and cathepsin. Therefore, Big-ET is useful not only as intermediate product to obtain pharmacologically active ET, but also as substrate to study and characterize enzymes involved in cell mediated ET synthesis.

At present Big-ET, as well as ET, are commercially available as direct chemical synthesis products from protected single amino acid residues.

Due to the complexity of ET molecules, having two disulfide bridges at 1-11 and 3-15 positions respectively, and to the peptide length, longer than the standard length of the usually synthesized peptides, the yield is low and, accordingly, costs result to be significatively high.

Genetic engineering methods allow to obtain recombinant peptides from genetically modified microorganisms at high yield and relatively low costs, provided that the concerned peptide is efficiently expressed in host cells and an effective purification procedure is available.

Both peptides and small protein molecules are generally present in few amount in bacterial cytoplasm, as being subjected to quick degradation. Recombinant molecules are subjected to similar degradation processes. Low molecular weigth recombinant peptides may efficiently be expressed as fusion products of heterologous high molecular weigth proteins, that result to be less sensitive to enzyme degradation. Then the fusion product is treated with specific enzymes, or suitable chemical reactants, which are able to release the peptide from the high molecular weigth protein.

A process to obtain Big-ET from engineered *E.coli* was described, using the approach as above, comprising a purifying process, wherein conventional chromatography separation techniques are used (Ohoshi and al., J. Biochem., 110, 628, 1991). According to this process, Big-ET was obtained as fusion product with β-galactosidase 55, 51 or 48 residue fragments, spaced with the Ile-Glu-Gly-Arg sequence, representing the cleavage site of the Xa enzyme (Pierce Chem. Co., USA) . The fusion product, which results to be poor soluble, is firstly purified from crude bacterial extracts through a gel filtration chromatography procedure, under denaturing conditions in presence of 8 M urea; then treated with the Xa enzyme, or trypsin, to release Big-ET. Big-ET is then purified by Reverse Phase High Pressure Liquid Chromatography (RP-HPLC). The method, that does not use any affinity purification step, is time consuming and can not be utilized to produce Big-ET on a large scale.

One of the authors of the instant invention has described (EP Application Nr.91830428.8) a peptide, named δ-ET(16-29), having the following L configuration amino acid sequence:
NH₂-ArgLysPheLeuAlaGlyLeuArgAlaArgArgLeuLysPhe-COOH, which is hydropathically complementary to the Big-ET 16-32 sequence, and able to bind the same both in liquid and covalently or not covalently solid phases. A synthesis process in a no linear form is described for said molecule, starting from an amino acid core, comprising at least one amino acid, having two aminic terminal functions, in order to facilitate its blocking on solid phase to prepare affinity columns. However, use of such columns to purify Big-ET from bacterial extracts, having proteolytic activities, is not feasible, as the immobilized ligand is easily degraded by enzymes contained in cell extracts, thus lowering the capacity and the functions of the column.

Accordingly, the availability of an easy, simple, high yield and cost effective process to obtain Big-ET, both for research and pharmacology purposes, is at present an actual requirement.

Surprisingly the authors have found that δ-ET ligand, even when synthesized using amino acids having a D configuration, is able to interact and recognize Big-ET. Further, said ligand, synthesized in a non linear multimeric form, is fully stable when subjected to proteolytic activities, such as trypsin, chymotrypsin and thermolysin, thus allowing to purify Big-ET directly from bacterial extracts, having high proteolytic activities. Therefore, recombinant Big-ET may be obtained directly from crude bacterial extracts as fusion product with other proteins and further digestion with suitable enzymes, through affinity chromatography columns with δ-ET as specific ligand. Big-ET is directly recovered by proteolytic extracts, so avoiding any intermediate steps, that could lower the final product yield. The method allows to obtain Big-ET at high yield also from partially or fully insoluble fusion products.

Accordingly, an object of the invention is a process to purify Big-ET from crude extracts, comprising the steps as follows:
- to express Big-ET protein in host cells using genetic engineering methods;
- to lyse said host cells and treat them with a proteolytic enzyme in order to have a proteolysed extract;
- to load said proteolysed extract to an affinity chromatography column, prepared by immobilizing a Big-ET specific ligand, said ligand comprising peptides hydropathically complementary to Big-ET, consisting of D configuration amino acids, said column equilibrated with a suitable saline buffer;
- to wash out the not specifically bonded material from said column by a suitable washing buffer;
- to elute the specifically retained material into said column by a suitable elution buffer.

Preferably, Big-ET is covalently bound to a molecule carrier, and has the formula:
(P)-X-R-(Big-ET)
wherein: P is said molecule carrier; X is a linear spacer peptide consisting from 1 to 10 amino acids; R is arginine; and Big-ET is the amino acid sequence of big endothelin.

According to a preferred embodiment of the invention, said hydropathically complementary peptides are non linear and comprise an amino acid core, having the following formula: 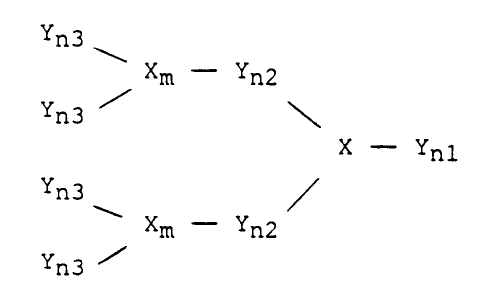 wherein: X is an amino acid having at least two amino terminal residues; Y is an amino acid selected from alanine, glycine or arginine; m is 0 or 1; n1, n2 and n3 are whole numbers from 0 to 10; and bonds are carbamide bonds.

According to a preferred embodiment of the invention, X is lysine or ornithine; more preferably m is 1.

Further according to the invention, said Big-ET hydropathically complementary peptides comprise the D configuration amino acid sequence as follows: NH₂-ArgLysPheLeuAlaGlyLeuArgAlaArgArgLeuLysPhe-COOH.

According to a another preferred embodiment of the invention, said non linear hydropathically complementary peptides have the following formula:

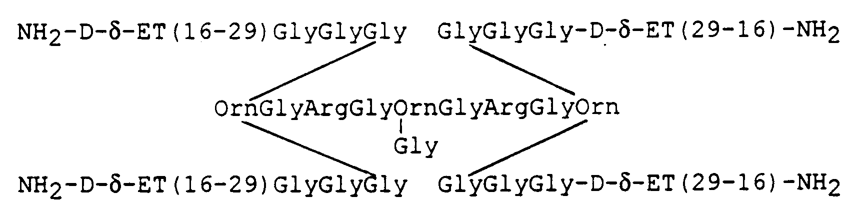

According to a preferred embodiment of the invention, said lysis is carried out by adding urea so that urea final concentration does not exceed 2 M, and the pH of the mixture is between 7 and 8.5; said proteolytic enzyme is selected from the group comprising trypsin, chymotrypsin and thermolysin.

This invention shall be described in the following by some explanatory, but not limiting examples, by reference to the annexed figures, wherein:
- fig.1 shows the resistance to proteolytic enzymes, as trypsin, chymotrypsin and thermolysin, of affinity chromatography ligand D-4-δ-ET;
- fig.2 shows the specificity of binding of Big-ET to affinity chromatography columns, prepared by immobilizing the ligand D-4-δ-ET;
- fig.3 shows trypsin digestion products of MBP-Big-ET fusion product;
- fig.4 shows the purification of the MBP-Big-ET, trypsin digested fusion product through an affinity chromatography procedure.

### EXAMPLE 1 GST-Big-ET and MBP-Big-ET fusion products

Genes coding the Maltose Binding Protein (MBP, 42 KD) (Duplay, P. and al., J. Biol. Chem., 259, 10606, 1984) and the Glutathione-S-Tranferase (GST, 27 KD) (Smith, D.B. and al., Proc. Nat'l. Acad. Sci. USA, 83, 8703, 1986) are used to construct chimeric coding sequences with Big-ET coding sequence. GST DNA coding sequence is in the pGEX-2T plasmid vector, as described by Smith, D.B. and al., Gene, 67, 31, 1988; while the MBP DNA coding sequence is in the pMal-CRI vector, as described in Di Guan, Gene, 67, 21, 1988; plasmids are available from Pharmacia and New England Biolabs, respectively. MBP-Big-ET and GST-Big-ET fusion genes are obtained using standard methods, as described in Sambrook, and al., Molecular Cloning: A Laboratory Manual, CSH Editor, 1989.

The human Big-ET coding sequence is obtained through *in vitro* assembling of three pairs of synthetic oligonucleotides. Each pair, formed by complementary oligonucleotides, is able to bind the adjacent pair by sticky ends. Therefore, three oligonucleotide pairs are covalently bound by T4 DNA ligase, producing the synthetic gene to be used as template in a gene amplification reaction, controlled by thermostable DNA Polymerase (PCR).

As PCR specific primers two oligonucleotides are used, having respectively a sequence complementary to the 5'end or to the 3'end of Big-ET and designed to insert DNA restriction sites to be used during the cloning step. Further, the 5'end oligonucleotide directs the insertion of the following four contiguous triplets ATCGACGGTCGT, coding the recognizing and cleavage site of Xa Factor, upstream the Big-ET coding sequence.

PCR obtained DNA fragments are purified by electrophoresis on agarose gel and cut by suitable restriction enzymes. Compatible restriction enzymes are used to cut pGEX-2T and pMal-CRI vectors. Ligation reactions covalently bind the vector and the fragment, thus producing two recombinant ET-GEX and ET-Mal2 plasmids.

*E. coli* cells (DH5α strain) are transformed with said plasmids by standard methods and recombinant clones are selected with ampicillin containing medium. Recombinant clones with a correct insertion of Big-ET at the 3'end of DNA of carrier proteins, are screened by restriction analysis and sequence identification, using the Sanger method (Sanger and al., Proc. Natl. Acad. Sci. USA, 74, 5463, 1977).

### EXAMPLE 2 Expression of GST-Big-ET and MBP-Big-ET fusion proteins

GST-Big-ET fusion protein is expressed by inducing ET-GEX plasmid with 1 mM isopropyl-β-D-thiogalactoside, when bacterial cultures have 0.72 absorbance units at 600 nm optical density.

MBP-Big-ET fusion protein is expressed by inducing Et-Mal2 plasmid with 0.3 mM isopropyl-β-D-thiogalactoside, when bacterial cultures have 0.45 absorbance units at 600 nm optical density.

The expression time, under optimized aeration and temperature (37°C) conditions, lasts three hours in both cases and is blocked by culture cooling and bacterial centrifugation. Bacterial pellets are washed using PBS phosphate buffer (0.2 g/l KCl; 0.2 g/l KH₂PO₄; 8 g/l NaCl; 1.15 g/l NaH₂PO₄). ET-GEX pellet is resuspended in one fifth of the initial volume of PBS, with 1% TRITON and 1 mM PMSF. ET-Mal2 pellet is resuspended in one twentyfifth of PBS initial volume, containing 0.25% hydrogenated TRITON and 1 mM PMSF.

Total bacterial protein extraction is carried out by ultrasonication. GST-Big-ET partially soluble fusion protein is localized mostly within the inclusion bodies, while MBP-Big-ET fusion protein is fully solubilized by bacterial lysis process.

### EXAMPLE 3 Evaluation of peptides' yield

Total bacterial lysate is subjected to SDS-PAGE under reducing conditions. Extract from ET-GEX induced bacteria contains appr. 25% of GST-Big-ET over the total amount of proteins, while extract from ET-Mal2 induced bacteria contains appr. 50% of MBP-Big-ET, over the total amount of proteins. Standard values of protein yield are in 0.1-0.6 mg/ml of culture range; the GST-Big-ET MBP-Big-ET yields are respectively 0.15 mg/ml and 0.3 mg/ml.

### EXAMPLE 4 Production and characterization of D-4-δ-ET peptide ligand

The sequence of hydropathically complementary peptide to human Big-ET (16-29) sequence, defined as δ-ET(16-29) is described in the European Patent Application N.91830428.8, as being NH₂-ArgLysPheLeuAlaGlyLeuArgAlaArgArgLeuLysPhe-COOH.

δ-ET(16-29) peptide is synthesized on solid phase, using standard Fmoc procedures, based on single amino acid coupling, that formed active esters with DCC/HOBt. All used amino acid residues in the synthesis have a D configuration, represented with "D". D-δ-ET (16-29) peptide is sinthesized in a tetrameric form (D-4-δ-ET), starting from a central core, consisting of ornithine-conjugated ornithine, according to the following scheme:

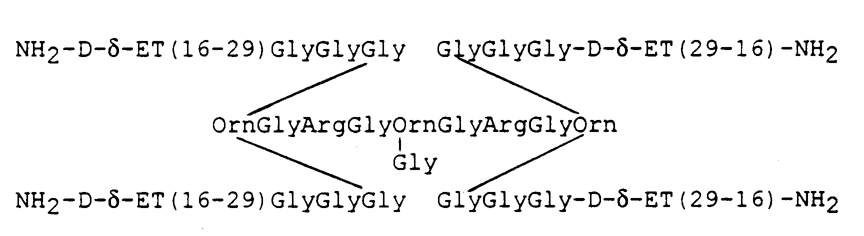

The multimeric peptide, defined as D-4-δ-ET(16-29), is deprotected, released from the resin by standard methods and then purified up to homogeneity by RP-HPLC. The purified peptide is analysed for amino acid content, as well for molecular weigth, by laser desabsorption mass spectrometry, thus confirming the peptide identity.

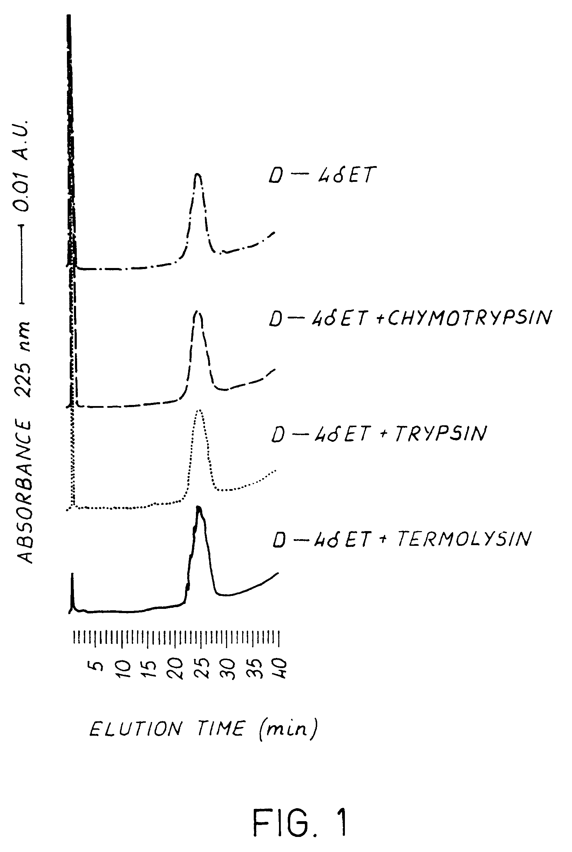

The purified peptide is dissolved at 100 µg/ml in 500 mM NH₄HCO₃ pH 8.0 and treated with trypsin, α-chimotypsin and thermolysin solutions, using an enzyme:substrate ratio 1:100 and incubating at 37°C. At different time intervals, aliquots are taken and analyzed by RP-HPLC for presence of degradation products. Fig.1 shows digestion data with different enzymes, confirming that the peptide is not degraded by any of tested enzymes, even after a four hour incubation.

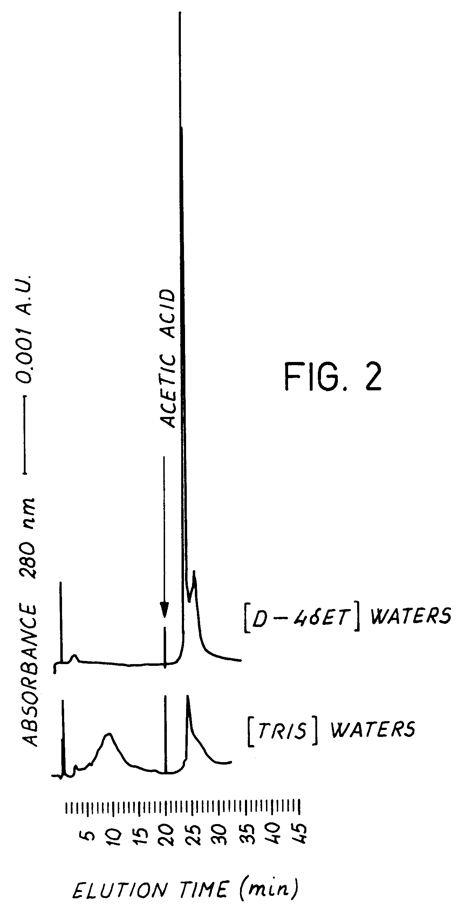

The peptide is used to prepare affinity columns. 5 mg of peptide dissolved in 10 ml NaHCO₃ are incubated with 1g of Eupergit C3ON (metacryl preactivated support with epoxide groups), or with 1,2 g of WATERS-ACCEL (coated siliceous support by a layer of polyacrylamide and preactivated with epoxide groups). After having been incubated with stirring for 24 hours at room temperature, resins are washed with coupling buffer and packed on I.D. 6.6 x 100 mm glass columns. Then columns, previously connected to a HPLC system, equipped with a variable wavelength detector and a recorder, are balanced with 25 mM TRIS, pH 6.8, at 2 ml/min flow rate. As preliminary step, the binding capacity to Big-ET is tested. For this purpose, 50 µl aliquots of 100 µg/ml solution of Big-ET in 25 mM TRIS pH 6.8, (NOVABIOCHEM, Switzerland) are injected into the columns and the eluent monitored by UV absorption at 280 nm. As shown in fig.2, the injected Big-ET sample does not elute from the column after 20 minutes, needing to replace the original buffer with an acetic acid buffer, to allow the sample to elute. The interaction between Big-ET and the blocked D-4 δ ET is specific as control columns, prepared by immobilizing unrelated peptides, or TRIS, show no ability to retain any Big-ET sample.

### EXAMPLE 5 Digestion of bacterial extracts containing MBP-Big-ET and GST-Big-ET fusion products

Bacterial extracts, prepared as in Example 1 and containing either Maltose Binding Protein-Big-ET or Glutathion Binding Protein-Big-ET fusion products, are 1:1 diluted with a 8 mM urea solution and incubated for 30 minutes at room temperature. Mixtures are 1:1 diluted with 50 mM NH₄HCO₃, pH 8.5 and treated with 10 µg trypsin per ml of mixture, under stirring conditions for 2 hours at room temperature. Extracts, which initially appear as insoluble materials, result as fully clarified and clear products.

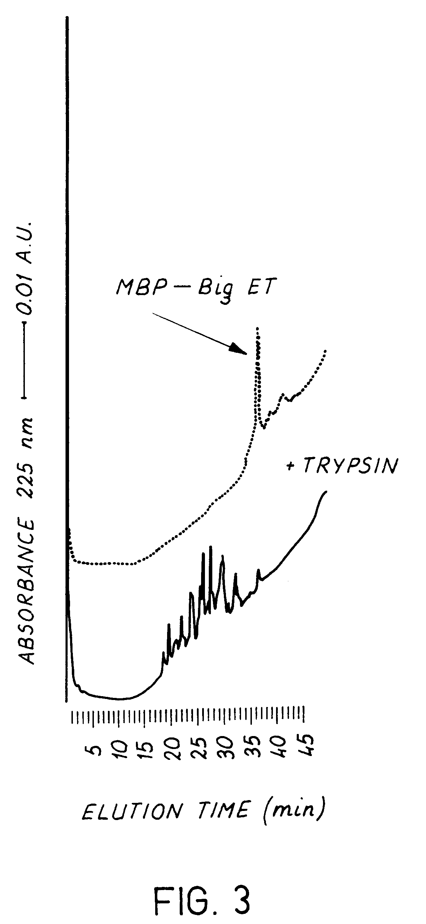

The fusion product cutting by trypsin digestion is checked by RP-HPLC, by monitoring the starting fusion product disappearing, as shown in fig.3 for MBP-Big-ET.

An Aquapore ABI column 2.1 x 30 mm I.D. is used in the separation chromatography step, which is balanced with 0.1% H₂O/CH₃/TFA, using a linear 3-60% acetonitrile gradient for 45 minutes. The column effluent is monitored by UV absorption at 225 nm. The process fully degrades the fusion product, thus producing a high Big-ET yield.

### EXAMPLE 6 Purification of Big-ET from trypsin treated bacterial extracts containing either MBP-Big ET or GST-Big-ET

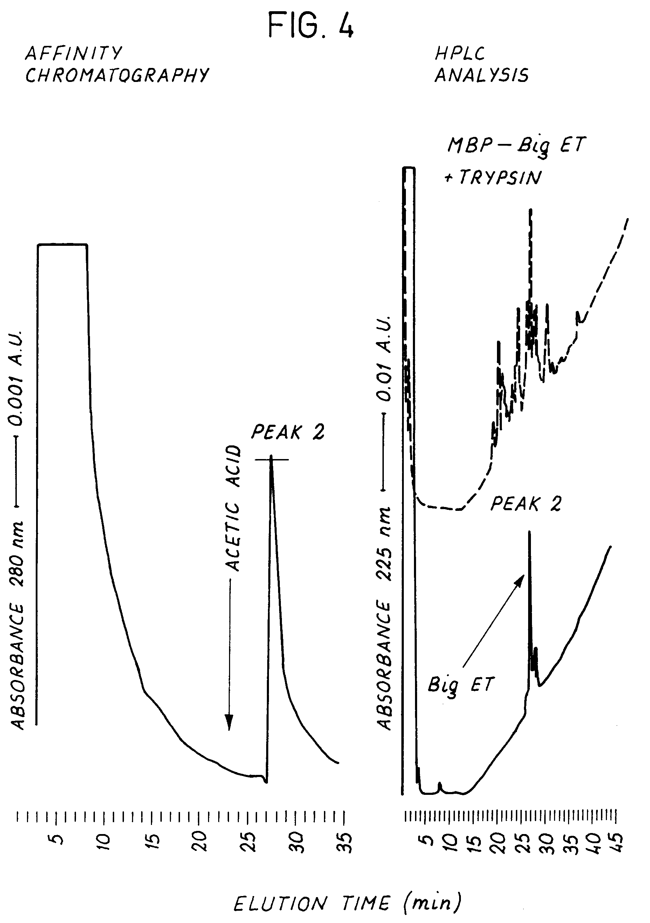

Bacterial extracts, treated as in Example 3, are 1:1 diluted with 10 mM TRIS pH 6.8 and 4 ml aliquots are loaded on either (D-4-δ-ET) Eupergit or (D-4-δ-ET) Waters affinity columns, balanced with 10 mM TRIS pH 6.8 at 1.0 ml/min. flow rate. After having fully eluted the column unretained material, the eluent is changed to 0.12 M acetic acid and the material collected and analyzed by RP-HPCL, as shown in fig.4 in the case of MBP-Big-ET.

It clearly results that both MBP-Big-ET and GST-Big-ET digested extracts are able to release Big-ET at high purity (80%) levels, by only one chromatography step. Quantitative measurements reveal that Big-ET is obtained at 20 µg/ml or 5 µg/ml, when respectively ET-2MAL or ET-GEX are used. Recovery yields are respectively 85% and 80%. The structural and sequence identity of purified material is tested by amino acid composition, as well as molecular weigth measurement through atomic bombardment mass spectrometry and chromatography retention time measurement on reverse phase columns.

The material purified by affinity chromatography on (D-4-δ-ET) Waters column is freeze dried, resuspended in 50 mM NH₄Ac, pH 7.5 buffer at 1 mg/ml concentration and treated with 20 µg α-chymotrypsin (SIGMA) by incubating with stirring for 2 hours at room temperature. Finally, the reaction mixture is analyzed by RP-HPCL. The mixture is freeze dried, added with 50:50:0.1 H₂O/CH₃CN/TFA up to a 5 mg/ml final concentration. Digestion products are separated by a semi-preparative RP-HPLC, using a RP I.D. 250 x 10 mm 8 Supelco column, equilibrated to a 3 ml/min. flow rate and a CH₃CN 5-65% linear gradient, in 45 minutes. Under the above conditions, endothelin elutes after 28 minutes, Big-ET after 32 minutes and the 22-38 fragment after 20 minutes. The endothelin based material is collected and freeze dried for subsequent use.

## Claims

1. Process to purify big endothelin (Big-ET) from crude extracts, comprising the steps as follows:
- to express Big-ET protein in host cells using genetic engineering methods;
- to lyse said host cells and treat them with a proteolytic enzyme in order to have a proteolysed extract;
- to load said proteolysed extract to an affinity chromatography column, prepared by immobilizing a Big-ET specific ligand, said ligand comprising Big-ET hydropathically complementary peptides, consisting of D configuration amino acids, said column equilibrated with a suitable saline buffer;
- to wash out the not specifically bonded material from said column by a suitable washing buffer;
- to elute the specifically retained material into said column by a suitable elution buffer.

2. Process to purify Big-ET from crude extracts according to claim 1, wherein said Big-ET is covalently bound to a molecule carrier, according to the formula:
(P)-X-R-(Big-ET)
wherein:wherein: P is said molecule carrier; X is a linear spacer peptide from 1 to 10 amino acids; R is arginine; and Big-ET is the amino acid sequence of the big endothelin.

3. Process to purify Big-ET from crude extracts according to any of previous claims, wherein said hydropathically complementary peptides are non linear and comprise an amino acid core, having the following formula: 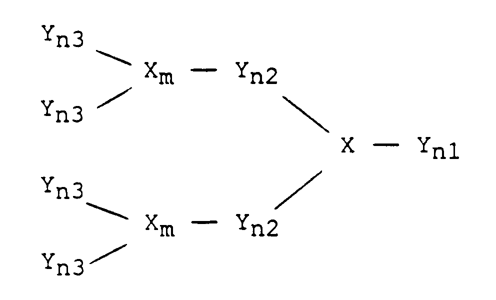 wherein: X is an amino acid having at least two amino terminal residues; Y is an amino acid selected from alanine, glycine or arginine; m is 0 or 1; n₁, n₂ and n₃ are whole numbers from 0 to 10; and bonds are carbamide bonds.

4. Process to purify Big-ET from crude extracts according to claim 3, wherein X is lysine or ornithine.

5. Process to purify Big-ET from crude extracts according to claims 3 or 4, wherein m is 1.

6. Process to purify Big-ET from crude extracts according to any of previous claims from 3 to 5, wherein said Big-ET hydropathically complementary peptides comprise the D configuration amino acid sequence as follows:
NH₂-ArgLysPheLeuAlaGlyLeuArgAlaArgArgLeuLysPhe-COOH.

7. Process to purify Big-ET from crude extracts according to claim 6 wherein said non linear hydropathically complementary peptides have the following formula:

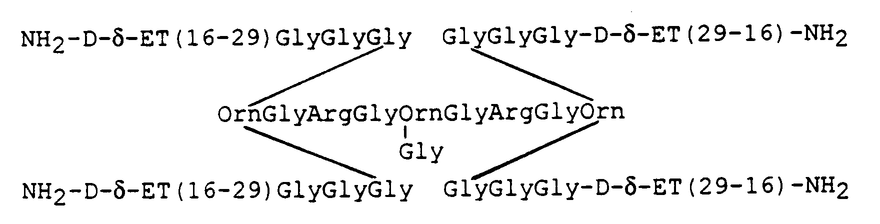

8. Process to purify Big-ET from crude extracts according to any of previous claim, wherein said lysis is carried out by adding urea so that urea final concentration does not exceed 2 M, and the pH of the mixture is between 7 and 8.5.

9. Process to purify Big-ET from crude extracts according to any of previous claim, wherein said proteolytic enzyme is selected from the group comprising trypsin, chymotrypsin and thermolysin.

## Patentansprüche

1. Verfahren zur Reinigung des Big-Endothelin Proteins aus Rohextrakten, bestehend aus folgenden Verfansschritten:
- Ausdrücken das Big-ET Protein in Wirtszellen durch Anwendung von Gentechnologie-Methoden;
- Lyse der vorgenannte Wirtszellen und Behandlung derselben mit einem proteolytischen Enzym zur Erhalung eines proteolysierten Extrakts;
- Laden des vorgenannten proteolysierten Extrakts in eine chromatographische Affinitätssäule, durch Immobilisierung von Big-ET spezifischen Liganden, wobei diese Liganden Big-ET hydropatisch komplementäre Peptiden enthalten, die aus D-formingen Aminosäuren bestehen und wobei die vorgenannte Säule mit einem entsprechenden Kochsalzpuffer ausgeglichen wird;
- Auswaschen des nicht spezifisch gebundenen Materials aus der vorgenannten Säule durch einen entsprechenden Washpuffer;
- Eluiren das spezifisch gebunde Material in der genannten Säule durch einen entsprechenden Elutionspuffer.

2. Verfahren zur Reinigung des Big-ET aus Rohextrakten nach Anspruch 1, worin das vorgenannte Big-ET mit einem Molekül-Träger kovalent gebunden ist und zwar gemäss der Formel:
(P)-X-R-(Big-ET),
worin P den vorgenannten Molekül-Träger darstellt;
X ein lineares Abstandhalter-Peptid mit 1 bis 10 Amino-Säuren ist; R ein Arginin ist und Big-ET eine Aminosäure-Sequenz des Big-Endothelins ist.

3. Verfahren zur Reinigung des Big-ET aus Rohextrakten nach nach je einem der vorhergehenden Ansprüche, worin die vorgenannten hydropatisch komplementäre Peptide nicht linear sind und einen Aminosäurekern Kern enthalten, der die folgende Formel hat: 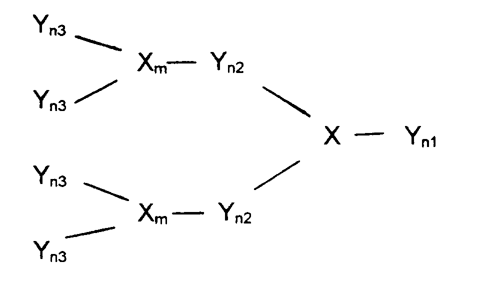 worin: X eine Aminosäure mit mindesten zwei Amino-Endresten ist; Y eine aus Alanin, Glycin oder Arginin ausgewählte Aminosäure darstellt; m gleich O oder 1 ist; n₁, n₂ und n₃ eine ganze Zahl von 0 bis 10 sind und Bindungen Karbamid-Bindungen sind.

4. Verfahren zur Reinigung des Big-ET aus Rohextrakten nach Anspruch 3, worin X Lysin oder Ornithin ist.

5. Verfahren zur Reinigung des Big-ET aus Rohextrakten nach Anspruch 3 oder 4, worin in gleich 1 ist.

6. Verfahren zur Reinigung des Big-ET aus Rohextrakten nach je einem der vorhergehenden Ansprüche 3 bis 5, worin die vorgenannten Big-ET hydropatisch komplementäre Peptide eine folgende D-förmige Aminosäuresequenz enthält;
NH₂-ArgLysPheLeuAlaGlyLeuArgAlaArgArgLeuLysPhe-COOH.

7. Verfahren zur Reinigung des Big-ET aus Rohextrakten nach Anspruch 6, worin die vorgenannten nicht linearen hydropathisch komplementären Peptide die folgende Formel haben:

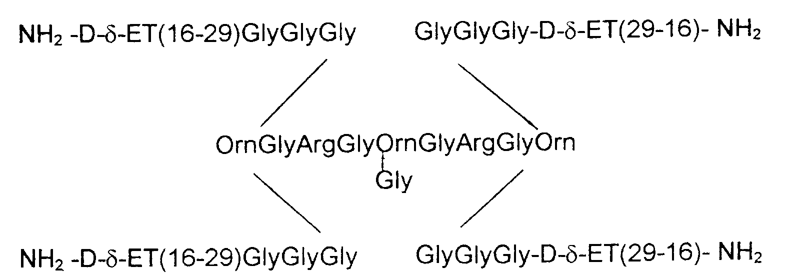

8. Verfahren zur Reinigung des Big-ET aus Rohextrakten nach nach je einem der vorhergehenden Ansprüche, worin die vorgenannte Lyse durch eine Zugabe von Harnstoff so durchgeführt wird, dass die Endkenzentration von Harnstoff 2M nicht überschreitet und der pH Wert des Gemisches zwischen 7 und 8,5 liegt.

9. Verfahren zur Reinigung des Big-ET aus Rohextrakten nach je einem der vorhergehenden Ansprüche, worin das vorgenannte proteolytische Enzym aud der Trypsin, Chymotrypsin und Thermosylin anthalten Gruppe ausgewählt wird.

## Revendications

1. Procédé de purification de protéine Big-endothéline des extraits bruts, comprenant les phases suivantes:
- exprimer le protéin Big-ET dans les cellules hôtes en utilisant des methodes de génie génétique;
- lyser lesdites cellules hôtes et traiter les mêmes avec une enzyme protéolytique afin d'obtenir un extract protéolysé;
- charger ledit extract protéolysé dans une colonne d'affinité chromatographique, préparée par l'immobilisation d'un ligant spécifique de Big-Et, ledit ligand comprenant des peptides hydrophatiquement complémentaires à Big-Et, consistants des aminoacides en conformation D, ladite colonne étant équilibrée par un convenable tampon salin;
- séparer par lavage le matériel non spécifiquement lié de ladite colonne par un convenable tampon de lavage;
- éluer le matériel spécifiquement retenu dans ladite colonne par un convenable tampon d'éluition.

2. Procédé de purification de protéine Big-endothéline des extraits bruts, selon la revendication 1, dans lequel ledit Big-Et est lié en convalence à une molécule carrier, selon la formule:
(P)-X-R-(Big-ET),
dans laquelle: P est ladite molecule carrier; X est un peptide entretoise linéaire de 1 à 10 aminoacides; R est arginine; et Big-Et est la séquence des aminoacides du big endothelin.

3. Procédé de purification de protéine Big-endothéline des extraits bruts, selon une quelconque des revendication précédentes, dans lequel lesdits peptides hydrophatiquement complémentaires ne sont pas linéaires et comprendent un noyau d'aminoacide, ayant la formule suivante: 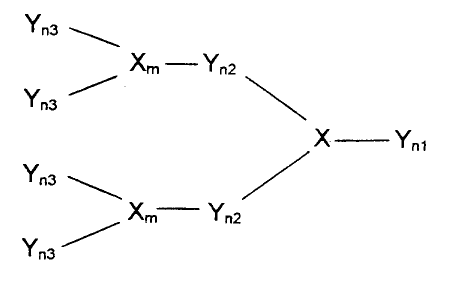 dans lequelle X est un aminoacide ayant au moins deux amino-résidu terminals; Y est un aminoacide choisi entre alanine, glycine ou arginine; un est 0 ou 1, n₁, n₂ et n₃ sont nombres entiers de 0 à 10 et les liaisons sont de liaisons carbamiques.

4. Procédé de purification de protéine Big-endothéline des extraits bruts, selon la revendication 3, dans lequel X est lysine ou omithine.

5. Procédé de purification de protéine Big-endothéline des extraits bruts, selon la revendication 3 ou 4, dans lequel m est 1.

6. Procédé de purification de protéine Big-endothéline des extraits bruts, selon une quelconque des revendications précédentes, dans lequel lesdits peptides hydropathiquement complémentaires comprendent la séquence des aminoacides en configuration D suivante:
NH₂-ArgLysPheLeuAlaGlyLeuArgAlaArgArgLeuLysPhe-COOH.

7. Procédé de purification de protéine Big-endothéline des extraits bruts, selon la revendication 6, dans lequel lesdits peptides hydropathiquement complémentaires ont la formule suivante:

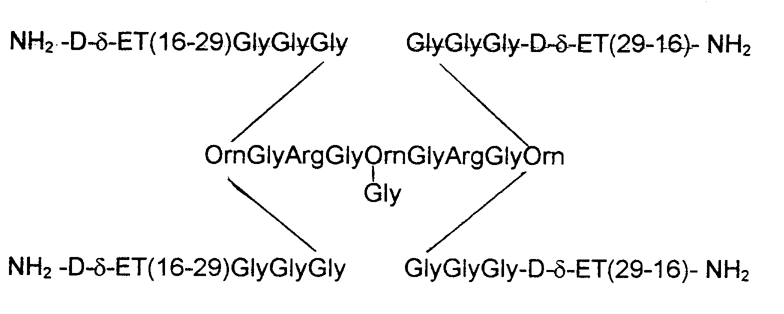

8. Procédé de purification de protéine Big-endothéline des extraits bruts, selon une quelconque des revendications précedéntes, dans lequel ladit lyse est réalisée par l'addition de urée de façon que la concentration finale d'urée ne dépasse pas 2M et le pH de la mélange est entre 7 et 8,5.

9. Procédé de purification de protéine Big-endothéline des extraits bruts, selon une quelconque des revendications précédentes, dans lequel ledit enzyme est choisi du groupe comprenant trypsin, chymotrypsin et thermolysin.
